# EUROPEAN PATENT SPECIFICATION

(11) **EP 1 827 338 B1**
(45) Date of publication and mention of the grant of the patent: **31.07.2013**
(21) Application number: 05854848.8
(22) Date of filing: 20.12.2005
(51) Int. Cl.: A61F 2/00

(54) **TREATMENT OF ANAL INCONTINENCE**
BEHANDLUNG ANALER INKONTINENZ
TRAITEMENT DE L'INCONTINENCE ANALE

(30) Priority: 20.12.2004 US 637665 P; 22.04.2005 US 673878 P
(43) Date of publication of application: 05.09.2007
(60) Divisional application: 11006532.3
(73) Proprietor: AMS Research Corporation, Minnetonka, MN 55343 (US)
(72) Inventor: Rosenblatt, Peter, L., MA 02465 (US)
(74) Representative: Bhimani, Alan
(86) International application number: PCT/US2005/046201
(87) International publication number: WO 2006/069078

(56) References cited:
- EP-A1- 0 639 355
- WO-A1-03/013392
- DE-C1- 19 544 162
- FR-A1- 2 852 817
- US-A- 3 789 828
- US-A1- 2004 039 453
- US-A1- 2004 215 054
- US-B2- 7 070 556

## Description

### BACKGROUND

Anal incontinence is a common problem that occurs in both men and women, though is certainly more prevalent in women after vaginal childbirth, presumably the result of trauma to pelvic floor muscles, supporting fascia and nerves. Fecal incontinence affects an estimated 7.6 percent of women between the ages of 30 - 90. The prevalence increases with age, affecting 3.6 percent of women between 30 - 39 and 15.2 percent of women between 80 - 90. Several factors contribute to anal continence, including the resting tone of the external and internal anal sphincters, as well as the position of the levator ani muscles, especially the puborectalis muscle, which forms a sling around the rectum and is responsible for the so-called "ano-rectal angle," which keeps stool in the rectum until voluntary defecation relaxes the puborectalis muscle and straightens the angle, allowing stool to move towards the anus.

Defecation is often aided by expulsive abdominal forces. Anal incontinence may occur as the result of several mechanisms, including direct damage to the internal or external anal sphincters (from iatrogenic episiotomy or spontaneous lacerations during vaginal delivery), or to the levator ani muscles. It may also result from indirect injury of these muscles through denervation of the nerves that supply these muscles. Treatment of this problem has centered on pelvic floor rehabilitation, dietary changes, or surgical correction. Surgery has been used to treat specific defects in the anal sphincters, such as external anal sphincteroplasty. Success rates of only 50% or less are generally reported for these procedures on long-term follow-up.

More recently, an artificial anal sphincter has been used to bypass these muscles, though this surgery involves fairly extensive dissection and requires the patient to depress a subcutaneous valve which temporarily deflates the sphincter cuff and allows voluntary defecation. This procedure is performed in very few centers in the U.S., and even in experienced hands, complications occur frequently. Dynamic graciloplasty, which involves mobilization and wrapping of the gracilis muscle around the anorectum is now another accepted procedure although is remains complex and requires extensive experience to obtain good results. More recently, sacral nerve stimulation has been used with some success to treat fecal incontinence, though the mechanism of success in these patients remains unclear, and may not be appropriate in women with obvious anatomic abnormalities, such as anal sphincter or levator muscle disruptions.

In addition, many women report other symptoms of bowel dysfunction, such as constipation and incomplete bowel emptying. For some women, these symptoms are due to either a anterior rectocele (a hernia of the rectum into the vaginal canal), or due to a defect in the levator ani muscles, which results in descent of the levator plate and / or perineum with abdominal straining. In addition, patients may be noted to have a defect in the posterior aspect of the rectum, or a posterior rectocele. There are very few treatment options for this condition, though retrorectal levatorplasty has been used in the past. In this procedure, an incision is made between the anus and the coccyx and the levator muscles are exposed bilaterally. Sutures are then placed in the levator muscles to plicate them together in the midline.
Active (inflatable) and passive Male and Female urethral prosthesis are known in the art, for example from US patent application 2004/0215054 (active) and US patent 3,789,828 (passive). These are surgically inserted into the patient to apply pressure to the urethra when normal muscular stoppage of urinary flow is not functional. US2004/0215054 discloses an inflatable pressure pad that can be inflated to block flow or deflated to allow flow and US 3,789,828 discloses a device that permanently applies resilient pressure to the urethra to block it. Flow is effected either by applying pressure via the bladder muscles of by manually pressing on the bladder to overcome the resilient pressure. US Patent Application 2004/0039453 discloses an implant for treating pelvic floor disorders having a central support are and attachment arms extending therefrom.

### SUMMARY

According to the present invention there is provided a rectal sling according to claim 1. Preferred features of the rectal sling are defined in claims 2 to 23.

### BRIEF DESCRIPTION OF THE DRAWINGS

FIG. 1 depicts the anatomy of the bony pelvis.

FIG. 2 depicts an exemplary placement of a device in the pelvis.

FIGS. 3-4 depict steps in one exemplary placement method.

FIGS. 5-6 depict steps in another exemplary placement method.

FIG. 7 depicts an exemplary final position of a length of supporting material.

FIGS. 8-10 depict exemplary uses of two lengths of supporting material.

FIGS. 11-19 depict exemplary uses of various instruments to position a length of supporting material.

FIGS. 20-22 depict exemplary slings.

FIGS. 23-25 depict exemplary slings having fluid-filled sacs.

FIGS. 26-29 depict an exemplary use of a stylet having a loop.

FIGS. 30-31 depict an exemplary use of a stylet having a hook.

FIGS. 32-38 depict additional exemplary embodiments of slings.

FIG. 39 depicts use of an exemplary device to measure the ano-rectal angle.

FIGS. 40-41 depict placement of a sling having a saddle-shaped central portion.

### DETAILED DESCRIPTION

The present disclosure provides a variety of systems and methods for treating anal incontinence. The normal ano-rectal angle can be restored by inserting a disclosed device under the posterior rectum, which may be supported with a synthetic or natural material in a sling-like position behind the anus and/or rectum. A posterior supporting apparatus may provide partial or complete closure of the rectum and/or anus with the posterior supporting apparatus.

In one embodiment, a synthetic or natural sling material may be placed under the rectum and may be supported by its arms, which may be extended up in a sling-like fashion through the obturator foramen bilaterally, or retropubically to the suprapubic region. In another embodiment, the device placed posterior to the rectum may include an inflatable or fluid-filled sac, which may or may not be adjusted post-operatively, by either changing its position or by altering the amount of fluid material, for example saline or a hydrogel, within the sac. "Fluid" is understood to include gasses, liquids, and semisolid media (such as gels). The central portion of the sling has a curved saddle shape, to help it conform to the external contour of the anus, rectum, ano-rectal angle, and/or levator ani muscles.

In a method of treating anal incontinence using the invnetion, an incision may be made between the anus and the coccyx and dissection performed, whereby the levator muscles and the levator plate are exposed. A small nick may be made on the medial thigh just lateral to the ischiopubic ramus and an introducer needle may be placed through the medial thigh incision, around the ischiopubic ramus, and be directed posteriorly into the ischiorectal fossa. The needle may then be directed lateral to the levator muscles, optionally with the assistance of a surgeon palpating the instrument though the vagina. The needle may then be brought posterior to the rectum, exiting the incision that was made in the midline. Alternatively, the needle may be passed from the midline incision between the anus and coccyx to the medial thigh incision lateral to the ischiopubic ramis. In one example, a suture may be threaded onto the eye of the needle, which in this case may be placed from the medial thigh to the incision between the anus and the coccyx, and may then be withdrawn through the tissue and held on the medial thigh. The procedure is repeated on the contralateral side. A synthetic (i.e., polypropylene, polyester, etc.) mesh (such as tension-free vaginal tape, TVT) or natural graft material may then be attached to each of the sutures coming from the midline incision, and the mesh may then be brought up through the medial thigh incisions by pulling up on the sutures.

The mesh may have a covering plastic sheath, which can facilitate passage through the tissues. The sheath may be removed when the sling is properly adjusted.

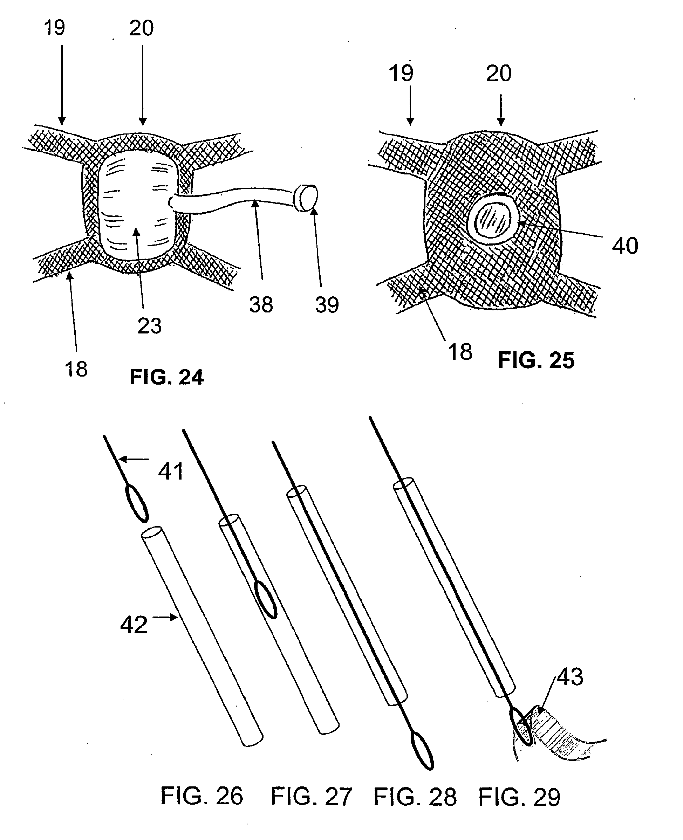

In another method of using the invention, the needle that is passed through the tissuc may have a hollow sleeve or tube over it (e.g., made of plastic, metal, or the like), and after passage, the needle may be withdrawn through the tissue, leaving the hollow sleeve in place. A stylet (e.g., made of plastic, metal, or the like) may then be then placed in the tube. The stylet may have a connector, such as a hook or a loop, so that a length of supporting material, such as a synthetic mesh (e.g., made of polypropylene or the like) or a natural graft, may be attached to the stylet connector. Exemplary uses of stylets with hooks or loops are shown in FIGS. 26-31. Once this procedure is performed bilaterally, the supporting material may be positioned under the rectum and the tension on the arms of the sling are adjusted. If the sleeves or tubes are utilized, the mesh can be adjusted before withdrawing the sleeves or tubes.

In another method of using the invention, two passes of the needles can be made on each side, one approximately at the level of the medial superior portion of the obturator foramen, and the other several centimetres inferior and slightly more lateral (at the inferior portion of the obturator foramen). This permits two lengths of supporting material (also called "mesh" but not necessarily limited to mesh) to be brought up on each side. These mesh strips are attached to a central mesh that may be placed under the rectum, which is a pre-formed mesh. The subrectal portion may be synthetic mesh, or may be made of another material, such as an inflatable or fluid-filled polymer sac. The sub-rectal element provides support to the posterior anus and/or rectum, and creates an angle between the anus and rectum, which keeps stool in the rectum until voluntary defecation.

In another method of using the invention, after the needle passes through the tissue, and is withdrawn, leaving a hollow tube in place, a plastic or metal stylet, previously fixed to the mesh with or without a sheath, can be placed up from the sub-rectal incision to the medial thigh incision and can be held. The mesh may then be brought up through the tubing by pulling on the stylet from above. Once the sling end comes out from the tubing, the hollow tube can be removed, after the sling has been adjusted for proper tensioning.

In another method of using the invention, the posterior aspect of the sub-rectal portion may be attached to the coccyx by one of several methods, such as direct suturing or with bone anchors. Such attachment can help maintain the position of the sub-rectal portion, which, in effect, restores the structure and function of the levator plate. Alternatively, the sub-rectal portion may have an extension coming off its inferior portion, which extends out and is fixed to the coccyx.

The fluid-filled sac under or adjacent to the rectum may have a port, such as a subcutaneous port, that may allow for fluid addition or removal in the post-operative period. This port may facilitate post-operative adjustment of the size and / or shape of the sac to provide for optimal results. The subcutaneous port may be placed directly under the sac, in the perineal skin, or may be connected to the sac by means of a connector tubing so that the port does not need to be located in the perineum itself, but instead may be positioned in a number of areas, including, for example, the buttocks.

The needle may have a hook near the end, that can be covered during insertion, but that may be exposed after the needle has been placed through the tissue. The user implanting the device may operate a switch or other actuator, such as a spring-loaded mechanism, to expose the hook. The arm of the sling, or a pre-loaded suture on the sling- arm, may then be placed on the hook and the needle withdrawn through the tissue.

In another method of using the invention, once the needle is placed through the tissue, the tip of the needle may be unscrewed off the end of the needle shaft. The arms of the sling may have a device attached to each end that may screw onto the needle shaft or otherwise fasten onto the needle shaft and then the needle is withdrawn, bringing the sling arm through the tissue.

A sheath covering the needle may remain in place in order to facilitate the movement of the synthetic material through the tissue, which is only removed once the tension on the sling is adjusted. The sheath may be defonnable, rather than rigid or semi-rigid, and may be flattened after removal of the needle, to accommodate the flat shape of the sling material itself.

The needle could have blunt metallic insert (to maintain the strength of the needle) with a plastic covering sheath that has a sharp needle tip configuration on the end. After the needle is placed through the tissue, the metallic blunt needle is withdrawn, and the plastic needle tip cut off. A suture retriever is then placed anterograde through the hollow plastic tube and grasps a suture that has been attached or pre-attached to the sling. The sling is withdrawn through the plastic tube and the tube is removed once the sling is adjusted.

In one example, the needle tip may be made from two separate pieces that act as jaws that open to catch the mesh or suture attached to the mesh, after the needle is passed through the tissue from the medial thigh to the incision posterior to the anus. This needle may be introduced with a plastic outer covering, so that the sling material may be drawn up through the tissue without catching on the surrounding structures. Once in proper position, the surgeon may remove the plastic sheath, which would then allow for the synthetic mesh to become fixed in the tissues.

A curved metal needle may be placed through the tissue from the medial thigh to the perineal incision. The end of the needle may be unscrewed, and the sling with attached plastic or metal piece may be screwed or snapped onto or into the connector on the needle. The sling, possibly with covering sheath, may then be withdrawn through the tissue and held and the plastic sheath is removed after the sling has been adjusted.

The shape of the sling may be a fixed width throughout its length. Alternatively, the central portion that is positioned under/behind the rectum may be wider than the arms. The central portion is curved to help it conform to the shape of the tissue it is supporting. The curved shape is a saddle shape, i.e. roughly a hyperbolic paraboloid or resembling a PRTNGLES(R) brand potato crisp. The central portion is preformed with the curved shape.

The mesh may be continuous throughout the length of the sling, or may have a central portion that includes a fluid-filled sac that is affixed to the sling anns on the sides. Preferably, the synthetic mesh would be continuous throughout its length in order to provide a backboard of support under the rectum and under the fluid-filled sac, if the fluid- filled sac is employed.

The fluid sac may have a circular or elongated shape under the rectum, or may include several compartments that can be separately filled with several access ports, in order to change the occlusion of the rectum. The fluid filled sac may have the curved shapes as discussed above.

Wings may connect a sling central portion to the arms of the sling. The wings may be made of mesh or other supporting material.

In another embodiment, the sling may be a hybrid of materials, comprised of, for instance, a polypropylene mesh along the arms of the sling in order to have self-attaching properties to the obturator fascia, and a natural xenograft material, such as porcine small intestinal submucosa, or an allograft, such as cadaveric fascia, located under and or lateral to the anus / rectum.

In another embodiment, the arms of the sling may include a synthetic material such as silastic or other plastic, and may have serrations that grab on to the obturator fascia as the arms are pulled through the tissue.

In another embodiment, the arms of the sling include sutures. There may be several sets of sutures on each side, in order to prevent the sub-rectal portion of the sling from rolling up underneath the anorectum.

In another embodiment, the arms of the sling may be attached to pelvic bone, such as the inferior-medial portion of the ischiopubic rami, or the inferior portion of the pubis, with bone anchors, suture material, or other fixation devices.

In another embodiment, the material under and / or lateral to the ano-rectum includes a synthetic material, such as silastic or other plastic material, that may be flexible, to conform to the shape of the bowel.

In another embodiment, a number of synthetic or natural elements are attached to the mesh in a direction transverse to the length of the sling, such as perpendicular or substantially perpendicular to the length of the sling. The elements may be semi-rigid and may be so positioned in the mesh as to be located under or lateral to the bowel when the mesh is deployed, for the purpose of keeping the mesh from rolling up underneath the anorectum. For example, the graft may have a stiff or flexible bar incorporated into the graft, located on either side of the rectum, to prevent rolling of the graft material.

In another embodiment, the sling may have additional straps attached to the subrectal portion that penetrate posteriorly, such as on either side of the coccyx, that may pass through the subcutaneous tissue and hold the graft in position, to prevent rolling of the subrectal mesh.

In another embodiment, the synthetic material may be elastic, which may permit stretching of the sling with abdominal straining, such as occurs with voluntary defecation.

In use, the sling may be passed through the levator ani muscle, rather than behind the muscles.

In another embodiment, not forming part of the claimed invention, the system may include a device used to evaluate the anorectal angle, for pre-operative diagnosis, intra-operative adjustment, and/or post-operative evaluation. The device is sufficiently flexible that it can be flexed to conform to the anorectal angle. The amount of flexion may be measured, thereby establishing the shape of the ano-rectal angle. In one example, the device may be inserted into the rectum and has a flexible joint which is placed at the junction between the anus and rectum. The device may then measure the angle created between the rectal and anal portions, and this angle may be displayed visually on the device, in one of a number of manners, including a dial or a digital display. The angle may also be communicated to an external display for convenience. The device may include a rotation- or position-sensitive transducer. This anorectal angle measurement device may be adapted so that is fits over the examiner's gloved finger with a portion that fits over or on the examiner's distal finger, and another portion that fits over or on the proximal finger. In this manner, when the examiner bends his or her finger to determine the ano-rectal angle, the measured angle is recorded visually on a display.

Various portions of the device may be coated, impregnated, or formed with one or more drugs to be eluted to an adjacent tissue. Various portions of the device may be formed of biodegradable or bioabsorbable material.

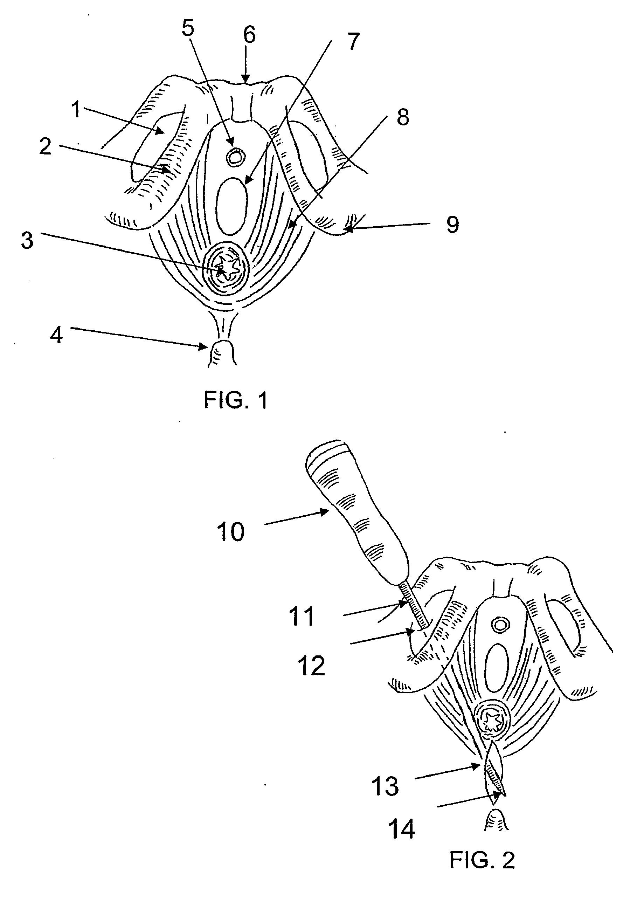

FIG. 1 shows the anatomy of the bony pelvis, with the pubic symphysis (6), the ischiopubic ramus (2), the ischial tuberosity (9), the coccyx (4), and the obturator foramen (I)- It also demonstrates the relationship of the levator ani muscles (and, in particular, the puhorectalis (8)) to the urethra (5), vagina (7), and rectum (3).

FIG. 2 demonstrates the placement of a needle (11) and attached handle (10) from the medial thigh incision (12), through the obturator membrane, into the ischiorectal fossa, and the needle tip (14) emerging through the vertical incision (13) between the anus and the coccyx.

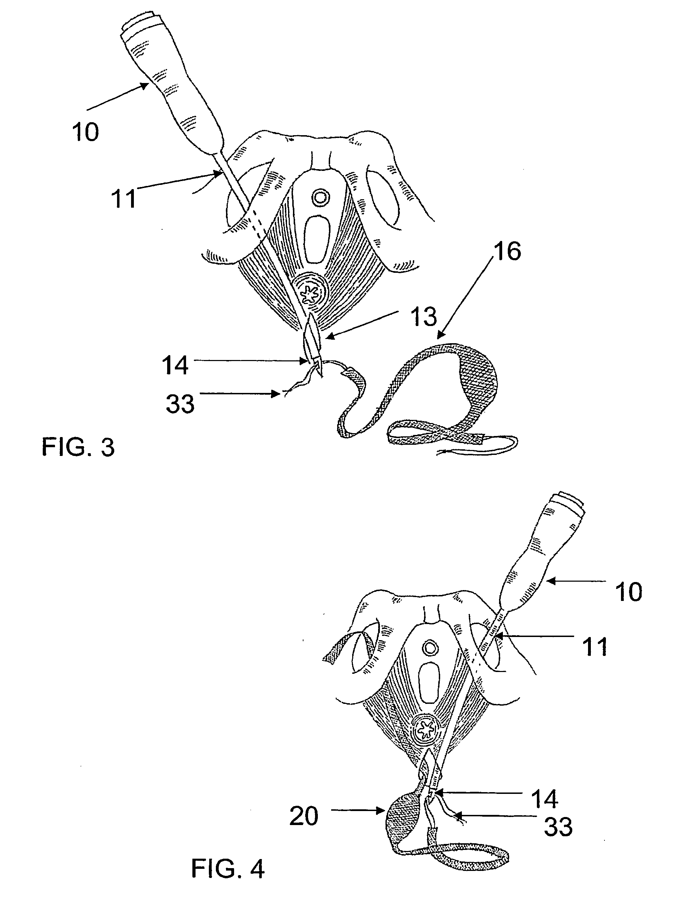

FIGS. 3-4 depict steps in one exemplary placement method. In FIG. 3, the needle (11) with the attached handle (10) has been placed through the thigh incision and through the obturator foramen, through the ischio-rectal fossa and out through the incision (13) between the anus and the coccyx. A suture loop (33) attached to the sling (16) is grasped by the needle tip (14) in order to transfer the sling to the thigh incision. **FIG. 4** demonstrates the second pass of the needle on the contralateral side with the handle (10) and needle (11) in place. The tip (14) is grasping the suture loop (33) in order to pull the other arm of the sling up through to the thigh incision. This allows the central portion of the sling (20) to rest under the ano-rectal area.

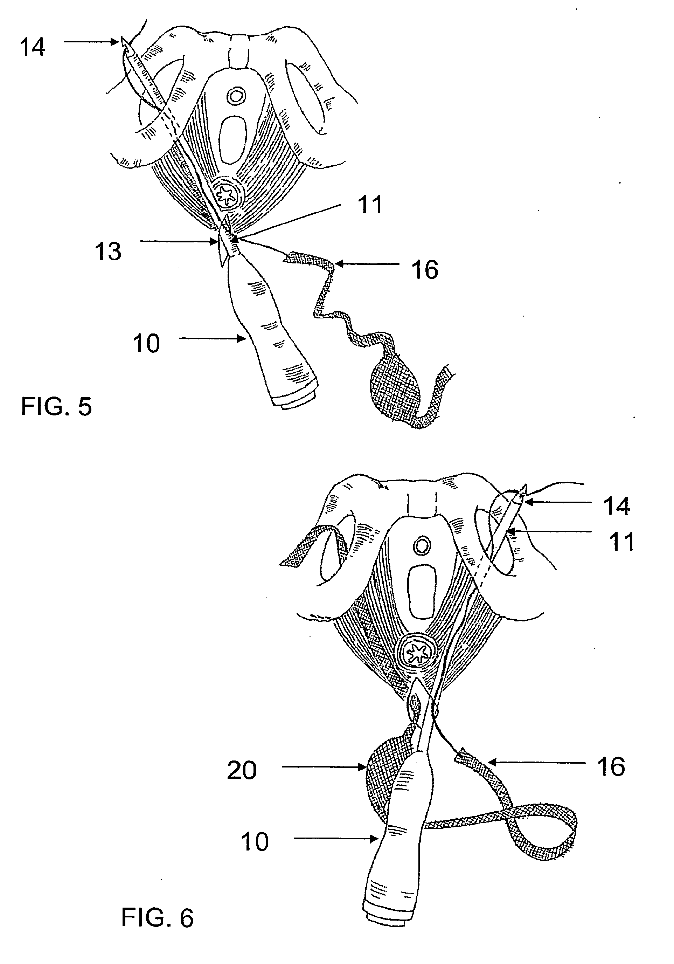

**FIGS. 5-6** depict steps in another exemplary placement method. In **FIG. 5****,** the needle (11) with attached handle (10) has been placed from the post-anal vertical incision (13) up through the ischiorectal fossa, through the obturator foramen, and out through the thigh incision, and has transferred a suture attached to the sling (18) to the thigh region. This allows the sling to be brought through the tissues up to the region of the thigh. **FIG. 6** shows the right side of the sling in place and the needle (11) with attached handle (10) transferring a suture attached to the left side of the sling arm (16) up through the left side. The suture is being held by the needle tip (14).

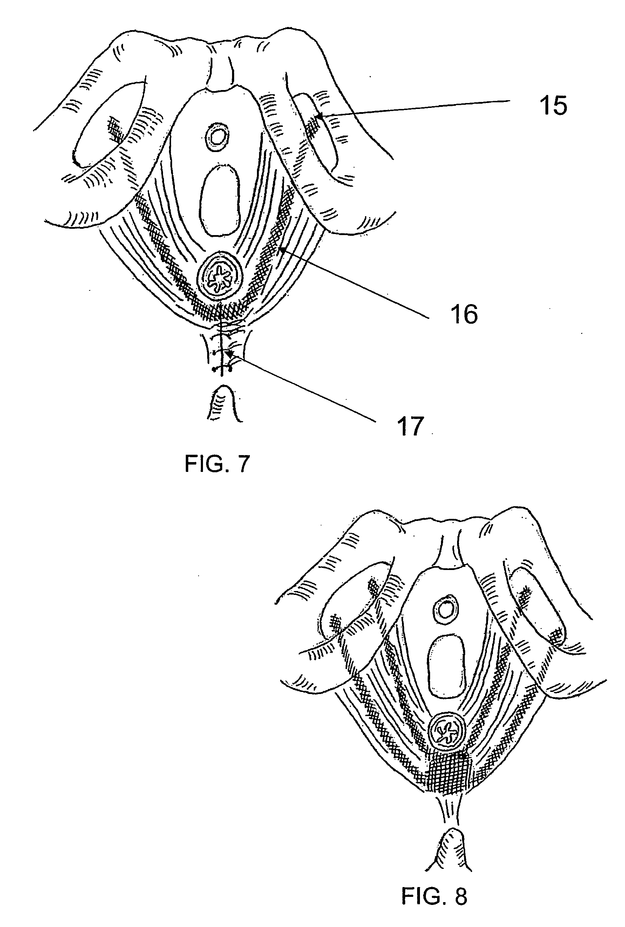

**FIG. 7** demonstrates a final position of the synthetic mesh (16) under the anus and/or rectum, with the incision between the anus and the coccyx (17), and up through the medial portion of the obturator membrane (15).

**FIG. 8** demonstrates the use of two synthetic mesh straps placed through the obturator membrane, the first more distal and placed near the superior-medial aspect of the obturator foramen (18), and the second placed near the inferior portion of the obturator foramen (19), and attached to a central element (20).

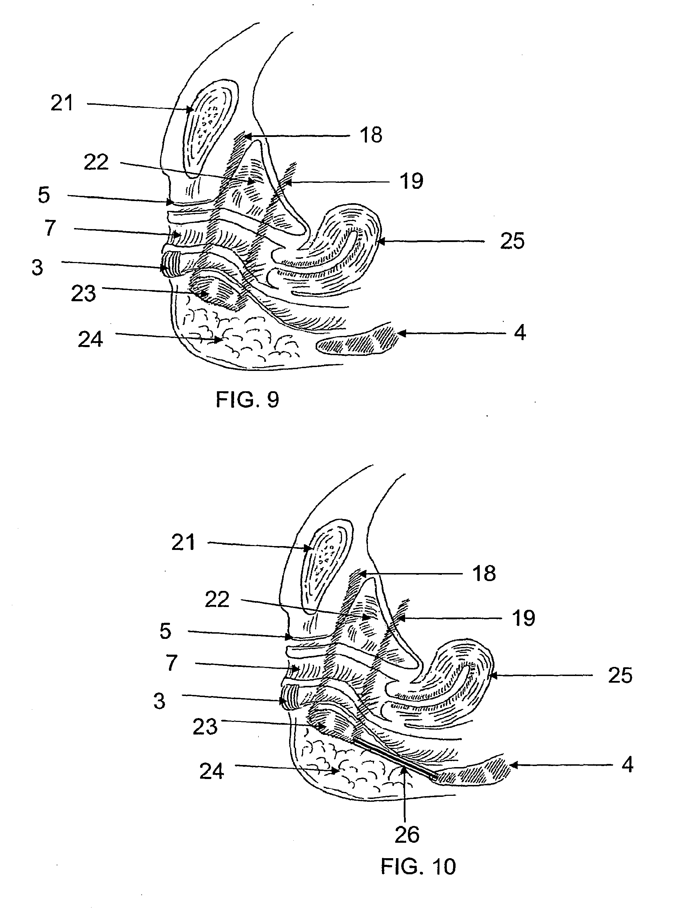

**FIG. 9** shows a lateral orientation of the pelvis with the pubic symphysis (21), the bladder (22), the uterus (25), and the ischiorectal fossa (24) with two synthetic straps on each side, the first more distal (18) and the other more proximal (19), and a sub-rectal element (23) that includes a fluid or gas filled reservoir.

**FIG. 10** shows a lateral orientation of the pelvis with the sling in place, with an extension (26) of the sub-rectal element attached to the coccyx with the use of sutures, bone anchor, or other method of affixing the synthetic material to the coccyx.

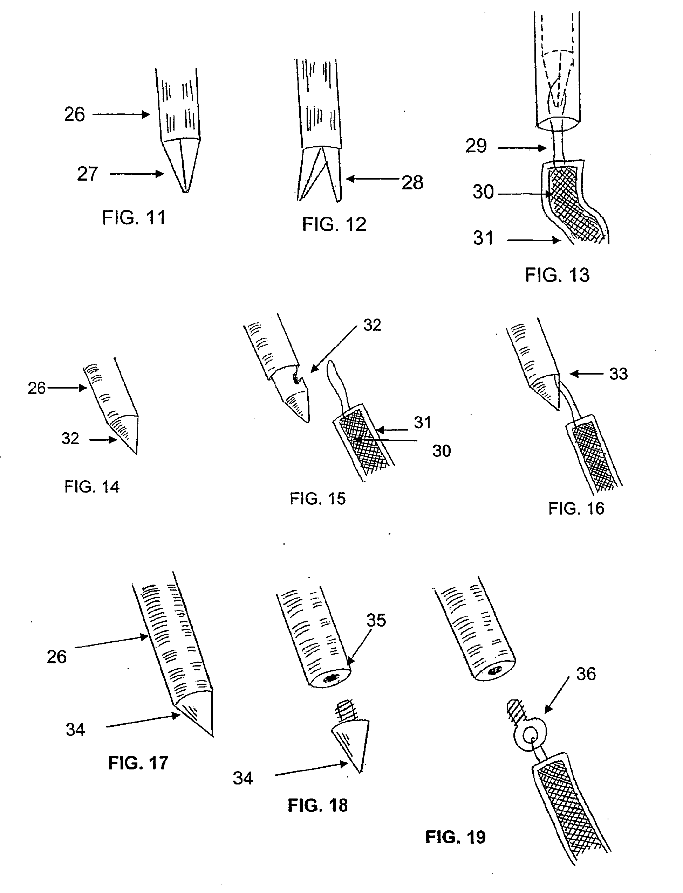

**FIGS. 11-13** demonstrate the use of a needle (27) introducer placed from the medial thigh to the incision under of the rectum. Once through the tissue, the jaw opens in the middle, which reveals a grasping instrument (28) that can hold on to a suture (29) affixed to the mesh (30) with or without a plastic sheath (31). The sling material is then brought through the tissue, with or without a plastic outer tubing (26) through which the grasper had been placed during the needle insertion..

FIGS. 14-16 demonstrate a needle (32) that, after insertion through the tissue, can be advanced beyond the outer sheath, with or without a spring-mechanism to deploy the needle. This reveals a notch, on which the suture loop (previously attached to the mesh), can be placed (33), and the sling (30) is then brought up through the tissues to the medial thigh

FIGS. 17-19 demonstrate a needle (34) that, after insertion through the tissue, may be separated from the shaft of the needle by unscrewing the needle tip. The sling would have a male-connector screw (36) that attaches to the straight needle shaft (35) and then the needle is withdrawn, which draws the mesh up through the tissue.

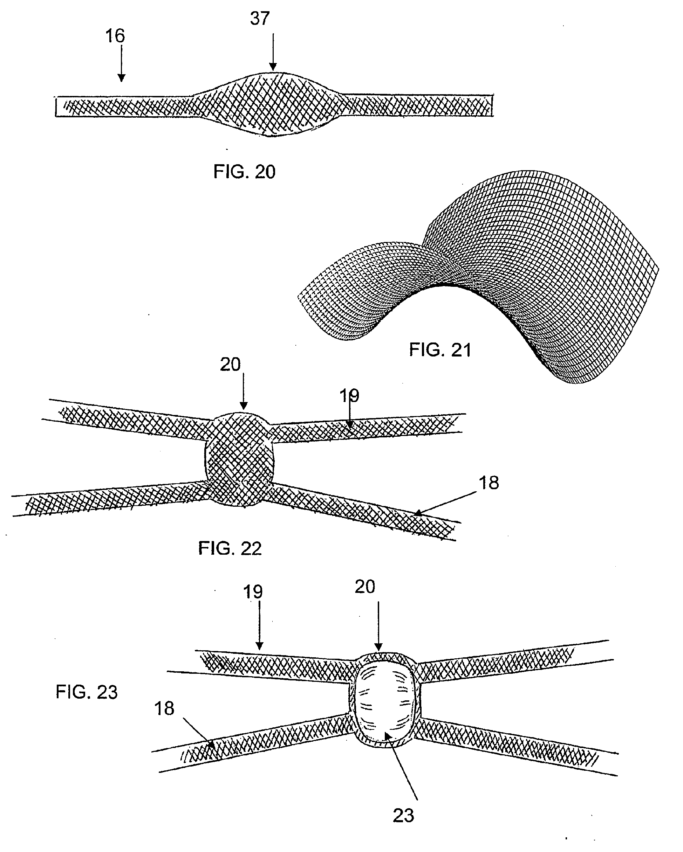

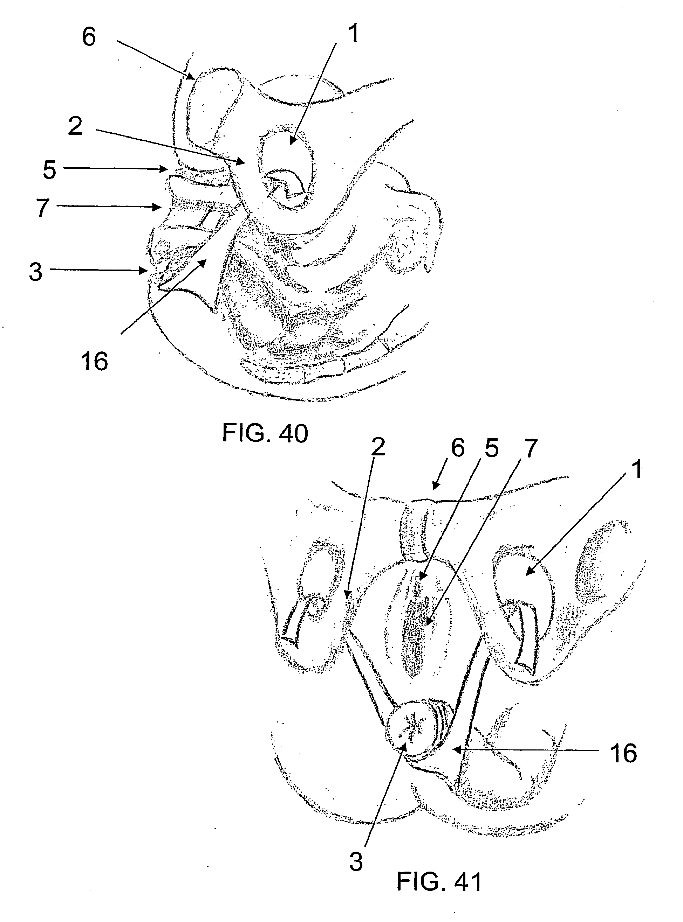

FIG. 20 demonstrates a sling that has narrow arms (16) and a wider area which would sit under the rectum (37) and distribute forces over a wide area. FIG. 21 illustrates a sling central portion (arms not shown) that has a curved shaped, specifically, a saddle shape. The saddle shape may facilitate making good contact with the anatomy to be supported. Its positioning is illustrated in FIGS. 40-41. One curve of the saddle allows the sling to arc between the obturator regions, while the other curve can complement the anorectal angle. FIG. 22 demonstrates an elongated central sling (20) with four attached arms, two of which are passed from the medial superior portion of the obturator membrane (18), and the other two which are passed through the inferior portion of the obturator membrane (19).

FIG. 23 demonstrates a superior view of a mesh that has a fluid-filled sac on the superior side of the graft material.

FIG. 24 shows another embodiment of a sling having a central portion with an inflatable sac. Connector tubing is attached to the fluid-filled sac and can be placed under the buttocks or other location within the reach of the tubing, and has a port at the end that can be used for filling or reducing the amount of fluid is contained within the sac.

FIG. 25 demonstrates an inferior view of the central portion (20), which shows a port from the fluid-filled sac coming out through a hole at the bottom of the graft (40). This port may be accessed subcutaneously in order to either add more fluid or remove fluid.

FIGS. 26-29 exhibit an exemplary use of a loop stylet. Stylet (41) may be advanced through tube (42). A length of sling material (43) may be threaded through the loop so that the material catches in the loop. The stylet may then be withdrawn back through the tube to bring the end of the sling material to the desired position.

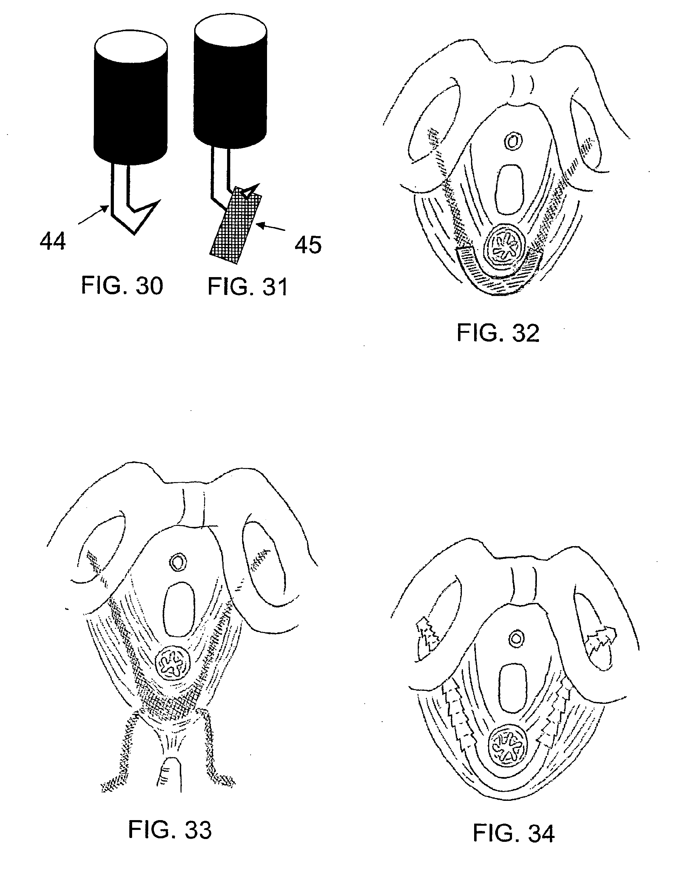

FIGS. 30-31 show an exemplary use of a hook stylet (44). A piece of sling material (45) may be stabbed onto a sharp tip of the hook. The hook may then be withdrawn through a tube to bring the end of the sling material to the desired location.

FIG. 32 shows a hybrid sling, comprised of, for instance, synthetic mesh arms attached to a central natural material placed under and / or lateral to the ano-rectum.

FIG. 33 shows the sling with additional straps of material that are attached to the subrectal portion of the device, and are secured in place by passage into subcutaneous tissue, in order to prevent rolling of the sling. The straps may be directed posteriorly, on either side of the coccyx, in order to keep the subrectal portion flat.

FIG. 34 demonstrates a sling made of a synthetic material such as silastic or other plastic with serrations on each arm that maintain the sling in position after adjustment by the surgeon

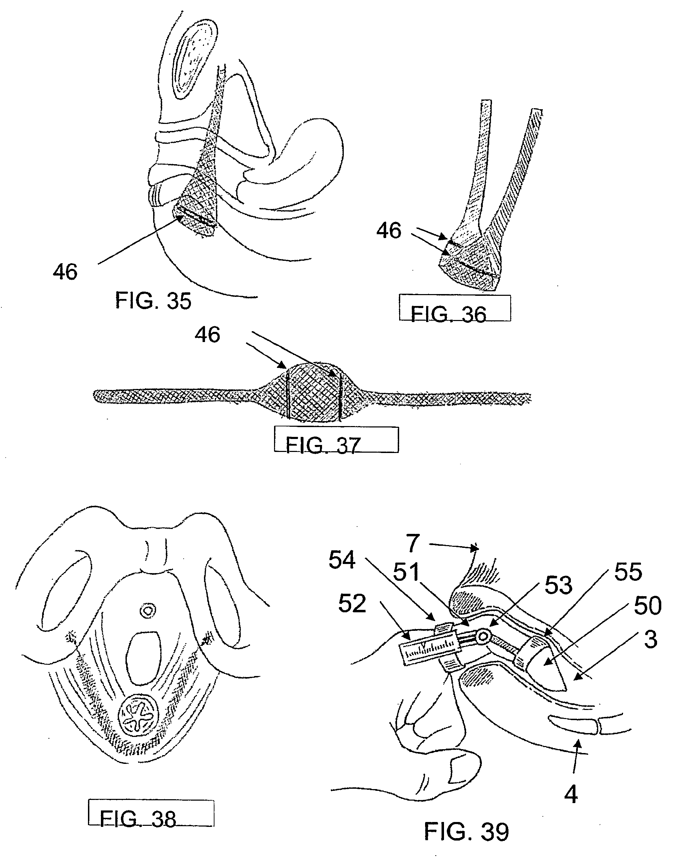

FIGS. 35-37 show embodiments of slings that include rigid or semi-rigid elements (46). The elements may be attached to the sling in order to keep the sling from rolling under the anorectal portion.

FIG. 38 shows the use of bone anchors to hold the sling into position, in this case to the inferior-medial portion of the ischiopubic rami.

FIG. 39 depicts a device, not forming part of the claimed invention, attached to the examiner's finger used to measure the angle between the rectum (3) and anus. The vagina (7) rests anterior to the anus and rectum, and the coccyx (4) is located posterior to the rectum. A proximal ring (54) is placed on the proximal phalanx (51) and the distal ring (55) is placed on the distal phalanx (50) and these are connected by a joint (53). The angle made between the anus and rectum is measured and displayed on a visual scale (52).

## Claims

1. A rectal sling (16) for reducing anal incontinence in a patient, the sling comprising a central portion (20) attached to at least two arms (18, 19) extending therefrom **characterized by**:
the central portion having a preformed saddle shape that is substantially a hyperbolic paraboloid that includes a first preformed curve that is structured to allow the sling to arc between obturator regions and a second preformed curve that is structured to complement the patient's ano-rectal angle.

2. The rectal sling (16) of claim 1, wherein each arm (18, 19) of the sling (16) is elastic so as to, in use, permit stretching of the sling with abdominal straining and the sling has a length sufficient to allow the arm (18, 19) to be extended, in use, through a respective obturator foramen.

3. The rectal sling (16) of claim 1, wherein each arm (18, 19) of the sling is elastic so as to, in use, permit stretching of the sling with abdominal straining and the sling has a length sufficient to allow the arm (18, 19) to be extended, in use, through a respective obturator membrane.

4. The rectal sling (16) of claim 1, wherein each arm (18, 19) of the sling is elastic so as to, in use, permit stretching of the sling with abdominal straining and the sling has a length sufficient to allow the arm (18, 19) to be extended, in use, to the subject's skin.

5. The rectal sling (16) of any preceding claim, wherein the first and second preformed curves of the central portion (20) allow the central portion (20) to conform to the shape of the tissue it is supporting.

6. The rectal sling (16) of any preceding claim, wherein the central portion (20) comprises an inflatable sac (23).

7. The rectal sling (16) of claim 6, wherein the sac (23) comprises a circular or elongate shape for location under the rectum.

8. The rectal sling (16) of claim 6 or 7, wherein the sac (23) has a port (39) for the introduction or removal of fluid.

9. The rectal sling (16) of claims 6 or 7 wherein the sac (23) comprises several compartments that can be separately filled via access ports so as to, in use, change the occlusion of the rectum.

10. The rectal sling (16) of any of claims 6 to 9 wherein the sac (23) is curved in shape.

11. The rectal sling (16) of any of claims 6 to 10 wherein at least one arm (18, 19) is formed of mesh.

12. The rectal sling (16) of any of claims 6 to 10 wherein at least one arm (18, 19) is formed of tension-free vaginal tape.

13. The rectal sling (16) of any of claims 6 to 12 wherein at least one arm (18, 19) terminates with a filament (29).

14. The rectal sling (16) of claim 13 wherein the filament (29) comprises a suture.

15. The rectal sling (16) of any one of claims 1 to 14 wherein the central portion (20) comprises a natural xenograft material or an allograft which, in use, is located under and/or lateral to the anus/rectum.

16. The rectal sling (16) of any one of claims 1 to 15 wherein the arms (18, 19) have serrations for grabbing the obturator fascia to, in use, maintain the central portion (20) in position after adjustment by a surgeon.

17. The rectal sling (16) of any one of claims 1 to 16 further including fixation devices for, in use, attachment of the arms (18, 19) of the sling to the pelvic bone.

18. The rectal sling (16) of claim 17 wherein the fixation devices comprise bone anchors attached to the arms (18, 19) at opposite ends thereof to the central portion.

19. The rectal sling (16) of any one of claims 1 to 18 wherein at least a portion of the sling is coated, impregnated or formed with one or more drugs to be, in use, eluted to adjacent tissue.

20. The rectal sling (16) of any preceding claim, wherein the arms (18, 19) are attachable to an end of a needle (11) thereby allowing, in use, the arms (18, 19) to be extended through an obturator foramen by pushing the needle (11).

21. The rectal sling (16) of claim 20, wherein each of the arms (18, 19) includes a filament (29) that is attachable to the end of said needle (11).

22. The rectal sling (16) of claim 21, wherein the filament (29) comprises a suture loop (33).

23. The rectal sling (16) of claim 22, further comprising said needle and wherein said needle (11) includes a needle tip (14) having one of: a jaw that opens in the middle to reveal a grasping instrument that can hold onto the suture loop or a notch on which the suture loop can be placed, so as to grasp the suture loop (33).

## Patentansprüche

1. Rektale Schlinge (16) für das Verringern der analen Inkontinenz bei einem Patienten, wobei die Schlinge einen mittleren Abschnitt (20) aufweist, der an mindestens zwei Armen (18, 19) befestigt ist, die sich von dort aus erstrecken, **gekennzeichnet dadurch, dass**:
der mittlere Abschnitt eine vorgeformte Sattelform aufweist, die im Wesentlichen ein hyperbolisches Paraboloid ist, das eine erste vorgeformte Kurve, die strukturiert ist, damit die Schlinge einen Bogen zwischen den Opturatorbereichen bilden kann, und eine zweite vorgeformte Kurve umfasst, die strukturiert ist, um den anorektalen Winkel des Patienten zu ergänzen.

2. Rektale Schlinge (16) nach Anspruch 1, bei der ein jeder Arm (18, 19) der Schlinge (16) elastisch ist, um so bei Benutzung das Strecken der Schlinge beim abdominalen Dehnen zu gestatten, und wobei die Schlinge eine Länge aufweist, die ausreichend ist, damit sich der Arm (18, 19) bei Benutzung durch ein jeweiliges Hüftbeinloch erstrecken kann.

3. Rektale Schlinge (16) nach Anspruch 1, bei der ein jeder Arm (18, 19) der Schlinge elastisch ist, um so bei Benutzung das Strecken der Schlinge beim abdominalen Dehnen zu gestatten, und wobei die Schlinge eine Länge aufweist, die ausreichend ist, damit sich der Arm (18, 19) bei Benutzung durch eine jeweilige Obturatormembran erstrecken kann.

4. Rektale Schlinge (16) nach Anspruch 1, bei der ein jeder Arm (18, 19) der Schlinge elastisch ist, um so bei Benutzung das Strecken der Schlinge beim abdominalen Dehnen zu gestatten, und wobei die Schlinge eine Länge aufweist, die ausreichend ist, damit sich der Arm (18, 19) bei Benutzung zur Haut des Patienten erstrecken kann.

5. Rektale Schlinge (16) nach einem der vorhergehenden Ansprüche, bei der die erste und zweite vorgeformte Kurve des mittleren Abschnittes (20) gestatten, dass sich der mittlere Abschnitt (20) an die Form des Gewebes anpasst, das er stützt.

6. Rektale Schlinge (16) nach einem der vorhergehenden Ansprüche, bei der der mittlere Abschnitt (20) einen aufblasbaren Beutel (23) aufweist.

7. Rektale Schlinge (16) nach Anspruch 6, bei der der Beutel (23) eine kreisförmige oder längliche Form für ein Anordnen unter dem Rektum aufweist.

8. Rektale Schlinge (16) nach Anspruch 6 oder 7, bei der der Beutel (23) eine Öffnung (39) für das Einführen oder Entfernen von Flüssigkeit aufweist.

9. Rektale Schlinge (16) nach Anspruch 6 oder 7, bei der der Beutel (23) mehrere Kammern aufweist, die separat über Zugangsöffnungen gefüllt werden können, um so bei Benutzung die Okklusion des Rektums zu verändern.

10. Rektale Schlinge (16) nach einem der Ansprüche 6 bis 9, bei der der Beutel (23) eine gebogene Form aufweist.

11. Rektale Schlinge (16) nach einem der Ansprüche 6 bis 10, bei der mindestens ein Arm (18, 19) aus Gaze geformt ist.

12. Rektale Schlinge (16) nach einem der Ansprüche 6 bis 10, bei der mindestens ein Arm (18, 19) aus spannungsfreiem Vaginalband geformt ist.

13. Rektale Schlinge (16) nach einem der Ansprüche 6 bis 12, bei der mindestens ein Arm (18, 19) mit einem Filament (29) endet.

14. Rektale Schlinge (16) nach Anspruch 13, bei der das Filament (29) ein Nahtmaterial aufweist.

15. Rektale Schlinge (16) nach einem der Ansprüche 1 bis 14, bei der der mittlere Abschnitt (20) ein natürliches xenogenes Transplantatmaterial oder ein allogenes Transplantat aufweist, das bei Benutzung unter dem und/oder seitlich des Anus/Rektums angeordnet wird.

16. Rektale Schlinge (16) nach einem der Ansprüche 1 bis 15, bei der die Arme (18, 19) Einkerbungen für das Erfassen der Opturatorfaszien aufweisen, um bei Benutzung den mittleren Abschnitt (20) nach der Regulierung durch einen Chirurgen in Position zu halten.

17. Rektale Schlinge (16) nach einem der Ansprüche 1 bis 16, die außerdem Befestigungsvorrichtungen für eine Befestigung der Arme (18, 19) der Schlinge am Beckenknochen bei Benutzung umfasst.

18. Rektale Schlinge (16) nach Anspruch 17, bei der die Befestigungsvorrichtungen Knochenanker aufweisen, die an den Armen (18, 19) an entgegengesetzten Enden davon zum mittleren Abschnitt befestigt sind.

19. Rektale Schlinge (16) nach einem der Ansprüche 1 bis 18, bei der mindestens ein Abschnitt der Schlinge mit einem oder mehreren Arzneimitteln beschichtet, imprägniert oder ausgebildet ist, die bei Benutzung am angrenzenden Gewebe eluiert werden.

20. Rektale Schlinge (16) nach einem der vorhergehenden Ansprüche, bei der die Arme (18, 19) am Ende einer Nadel (11) befestigt werden können, wodurch bei Benutzung gestattet wird, dass die Arme (18, 19) durch ein Hüftbeinloch gezogen werden, indem die Nadel (11) geschoben wird.

21. Rektale Schlinge (16) nach Anspruch 20, bei der ein jeder der Arme (18, 19) ein Filament (29) umfasst, das am Ende der Nadel (11) befestigt werden kann.

22. Rektale Schlinge (16) nach Anspruch 21, bei der das Filament (29) eine Nahtmaterialschlinge (33) aufweist.

23. Rektale Schlinge (16) nach Anspruch 22, die außerdem die Nadel aufweist, und wobei die Nadel (11) eine Nadelspitze (14) umfasst, die eines von Folgendem aufweist:
eine Klemmbacke, die sich in der Mitte öffnet, um ein Greifinstrument zu zeigen, das die Nahtmaterialschlinge halten kann; oder eine Kerbe, in der die Nahtmaterialschlinge angeordnet werden kann, um so die Nahtmaterialschlinge (33) zu ergreifen.

## Revendications

1. Bandelette rectale (16) pour déduire l'incontinence anale d'un patient, la bandelette comprenant une partie centrale (20) fixée sur au moins deux bras (18, 19) s'étendant à partir de celle-ci, **caractérisée en ce que** :
la partie centrale a une forme en selle préformée, constituée pratiquement par un paraboloïde hyperbolique englobant une première courbe, structurée de sorte à permettre la formation d'un arc par la bandelette entre des régions obturatrices, et une deuxième courbe préformée, structurée de sorte à compléter l'angle ano-rectal du patient.

2. Bandelette rectale (16) selon la revendication 1, dans laquelle chaque bras (18, 19) de la bandelette (16) est élastique, de sorte à permettre en service un étirement de la bandelette en présence d'une tension abdominale, la bandelette ayant une longueur suffisante pour permettre en service l'extension du bras (18, 19) à travers un trou obturateur respectif.

3. Bandelette rectale (16) selon la revendication 1, dans lequel chaque bras (18, 19) de la bandelette est élastique, de sorte à permettre en service un étirement de la bandelette en présence d'une tension abdominale, la bandelette ayant une longueur suffisante pour permettre en service l'extension des bras (18, 19) à travers une membrane obturatrice respective.

4. Bandelette rectale (16) selon la revendication 1, dans laquelle chaque bras (18, 19) de la bandelette est élastique, de sorte à permettre en service un étirement de la bandelette en présence d'une tension abdominale, la bandelette ayant une longueur suffisante pour permettre en service l'extension du bras (18, 19) sur la peau du sujet.

5. Bandelette rectale (16) selon l'une quelconque des revendications précédentes, dans laquelle les première et deuxième courbes préformées de la partie centrale (20) permettent l'adaptation de la partie centrale (20) à la forme des tissus qu'elle supporte.

6. Bandelette rectale (16) selon l'une quelconque des revendications précédentes, dans laquelle la partie centrale (20) comprend un sac gonflable (23).

7. Bandelette rectale (16) selon la revendication 6, dans laquelle le sac (23) a une forme circulaire ou allongée en vue d'un positionnement au-dessous du rectum.

8. Bandelette rectale (16) selon les revendications 6 ou 7, dans laquelle le sac (23) comprend un orifice (39) pour l'introduction ou le retrait d'un fluide.

9. Bandelette rectale (16) selon les revendications 6 ou 7, dans laquelle le sac (23) comprend plusieurs compartiments pouvant être remplis séparément à travers des orifices d'accès, de sorte à changer en service l'occlusion du rectum.

10. Bandelette rectale (16) selon l'une quelconque des revendications 6 à 9, dans laquelle le sac (23) a une forme courbée.

11. Bandelette rectale (16) selon l'une quelconque des revendications 6 à 10, dans laquelle au moins un bras (18, 19) est formé de maille.

12. Bandelette rectale (16) selon l'une quelconque des revendications 6 à 10, dans laquelle au moins un bras (18, 19) est formé par une bande vaginale exempte de tension.

13. Bandelette rectale (16) selon l'une quelconque des revendications 6 à 12, dans laquelle au moins un bras (18, 19) se termine par un filament (29).

14. Bandelette rectale (16) selon la revendication 13, dans laquelle le filament (29) comprend une suture.

15. Bandelette rectale (16) selon l'une quelconque des revendications 1 à 14, dans laquelle la partie centrale (20) comprend un matériau de xénogreffe naturel ou une allogreffe, agencée en service au-dessous de l'anus/du rectum ou/ou latéralement par rapport à ceux-ci.

16. Bandelette rectale (16) selon l'une quelconque des revendications 1 à 15, dans laquelle les bras (18, 19) comportent des dentelures pour saisir le fascia obturateur, en vue de maintenir en service la partie centrale (20) dans sa position après l'ajustement par un chirurgien.

17. Bandelette rectale (16) selon l'une quelconque des revendications 1 à 16, englobant en outre des dispositifs de fixation pour fixer en service les bras (18, 19) de la bandelette sur l'os iliaque.

18. Bandelette rectale (16) selon la revendication 17, dans laquelle les dispositifs de fixation comprennent des ancrages des os fixés sur le bras (18, 19) au niveau de ses extrémités opposés à la partie centrale.

19. Bandelette rectale (16) selon l'une quelconque des revendications 1 à 18, dans laquelle au moins une partie de la bandelette est revêtue, imprégnée ou formée avec un ou plusieurs médicaments devant être élués en service vers les tissus adjacents.

20. Bandelette rectale (16) selon l'une quelconque des revendications précédentes, dans laquelle les bras (18, 19) peuvent être fixés sur une extrémité d'une aiguille (11), permettant ainsi en service l'extension des bras à travers un foramen obturateur en poussant l'aiguille (11).

21. Bandelette rectale (16) selon la revendication 20, dans laquelle chacun des bras (18, 19) englobe un filament (29) pouvant être fixé sur l'extrémité de ladite aiguille (11).

22. Bandelette rectale (16) selon la revendication 21, dans laquelle le filament (29) comprend une boucle de suture (33).

23. Bandelette rectale (16) selon la revendication 22, comprenant en outre ladite aiguille, ladite aiguille (11) englobant une pointe d'aiguille (14) comportant un des éléments ci-dessous : une mâchoire s'ouvrant au milieu pour révéler un instrument de préhension pouvant retenir la boucle de suture, ou une encoche sur laquelle la boucle de suture peut être placée, de sorte à saisir la boucle de suture (33).
